# EUROPEAN PATENT SPECIFICATION

(11) **EP 3 156 761 B1**
(45) Date of publication and mention of the grant of the patent: **30.12.2020**
(21) Application number: 15189853.3
(22) Date of filing: 14.10.2015
(51) Int. Cl.: G01B 11/00, A61B 1/00, B25J 19/02

(54) **IMAGE ACQUISITION AND PROCESSING**
AUFNAHME UND VERARBEITUNG EINES BILDES
ACQUISITION ET TRAITEMENT D'IMAGE

(43) Date of publication of application: 19.04.2017
(73) Proprietor: H4 Technologies Limited, Buckinghamshire HP15 6TL (GB)
(72) Inventor: HAYFORD, Paul Derek, High Wycombe, Buckinghamshire HP15 6TL (GB); HAYFORD, Joshua Paul, High Wycombe, Buckinghamshire HP15 6TL (GB)
(74) Representative: Faulkner, Thomas John

(56) References cited:
- WO-A-91/11290
- WO-A1-2013/025530
- WO-A1-2015/080669
- DE-A1-102012 203 134
- US-B1- 6 503 195

## Description

This invention relates to image acquisition and processing methods and apparatus which are arranged for determining positional information in respect of an object in an object area.

Such apparatus and methods may be useful in different circumstances. Thus, for example image acquisition and processing apparatus which can be used for determining positional information in respect of an object may be useful in areas such as multi degree of freedom robots, conveyor systems, image processing assisted surgery, robotic surgery and strain field measurement. These are all systems where images of an object in an object area are acquired and processed and the processed images put to some further use. In some circumstances the processed images may be displayed to a user or in more complex systems the processed images may be used to directly control apparatus.

Thus, in more general terms, digital images, that is, single digital frames or video images can be used in manufacture, quality control, and measurement systems provided that appropriate signal processing and analysis is provided to allow the desired production of images and to control decisions and so on.

As is well understood, signal processing systems can make use of a large range of techniques to interpret information. These include pattern recognition, measurement algorithms, edge detection, and image correlation. In at least some circumstances, the signal processing techniques can be unreliable due to unwanted image artefacts created by lighting arrangements and/or due to external light interference. In particular light reflections (that is specular reflections) can significantly change the image, upset the contrast level and make important details unrecognisable.

Therefore it would be desirable to provide a system which can reduce the occurrence and/or effect of such image artefacts. Further it is particularly desirable if the resulting system offers a high degree of flexibility in the circumstances in which it can be used and/or the set up of the system.

DE 10 2012 203 134 describes a method for recording a contour part by means of at least one manipulator and a device for receiving 5 a contour part, comprising at least one manipulator, at least one sensor device and at least one control device.

US 6,503,195 describes a real-time structured light depth extraction system including a projector for projecting structured light patterns onto an object of interest.

WO 2013/025530 describes a minimally invasive surgical system having an image capture unit including a lens assembly and a sensor assembly.

WO 2015/080669 describes a flexible vision inspector comprising a first type of illuminator for illuminating an object, a second type of illuminator for projecting light onto the object, and a housing.

WO 91/11290 describes an imaging device suitable for use in guiding a robot arm supporting a welding torch in automatic welding applications carried out by welding robots.

According to one aspect of the invention there is provided an object handling apparatus comprising a surgical robot having a robotic arm and further comprising image acquisition and processing apparatus arranged for determining positional information in respect of an object area occupied by an object, the apparatus comprising:
a light source arrangement for illuminating the object area;
a light detector arrangement for detecting light reflected from the object area; and
image processing means arranged for determining positional information in respect of the object area in dependence on output from the light detector arrangement;
a control unit arranged for controlling the robotic arm in dependence on the output from the image processing means;
wherein the light source arrangement is arranged for illuminating the object area with elliptically polarised light with a first chirality; and the light detector arrangement comprises an elliptical polarisation filter for allowing passage of elliptically polarised light with said first chirality and blocking elliptically polarised light with an opposite chirality for suppressing generation of image artefacts due to specular reflection of the elliptically polarised light from the object area and wherein the robotic arm comprises an object manipulation member provided in the object area for manipulating the object, the object manipulation member having a surface which diffusely reflects light from the light source.

Image artefacts due to specular reflection can obscure image features (such as edges of an object), the detection of which are commonly used in image processing to recognise an object and hence determine its position. Similarly any registration marks or equivalent provided on an object to help identify it and/or its position can be obscured by image artefacts due to specular reflection. The chirality (or handedness - ie direction of E-vector rotation) of elliptically polarised light is reversed on pure specular reflection. Thus illumination by elliptically polarised light and the use of an appropriate elliptical polarisation filter in the detection system can help eliminate such artefacts. On the other hand of course, diffusely reflected light without such polarisation will be substantially unaffected by the filters and can reach the light detection system.

Typically the elliptically polarised light will be circularly polarised. However the system can function well with other non-linearly polarised, elliptically polarised light.

This is because with elliptically polarised, say circularly polarised, light, the system can be fairly insensitive to angular and positional set up of the light source arrangement, the light detector arrangement and the object. The chirality of the light is well preserved and the filtering still effective for reduction of unwanted image artefacts even as such angles and positions change. This is not the same for a system which relies on linearly polarised light. There much more precise alignment of the components is required to achieve good results.

Thus the present techniques can provide easier set up and greater versatility than ones relying on linearly polarised light.

In general the present techniques may be used with multi-degree of freedom motion equipment with imaging systems. Such equipment may be used in medical applications (including image processing assisted surgery and/or robotic surgery).

The apparatus may be image processing assisted surgery apparatus. In use of such apparatus, images acquired by the apparatus and enhanced by the apparatus are displayed to a user -ie surgen. Note that in surgery fluid present in the field of view can be particularly problematic in generating reflection artefacts and ones that tend to change with time. The present techniques can significantly dampen the effects of these.

The object handling apparatus may comprise control means arranged to make decisions and/or control components in dependence on output from the light detector arrangement in particular an output from the image processing means. A decision may, for example, be to accept or reject an object.

The object handling apparatus may comprise control means for controlling the position of the object manipulation member in the object area. The control means may be arranged for controlling the position of the object manipulation member in dependence on output from the light detector arrangement.

The object handling apparatus may comprise a plurality of object manipulation members. The control means may be arranged for controlling the position of each of the object manipulation members in dependence on output from the light detector.

The object may be such as to have a surface which diffusely reflects light from the light source.

The object manipulation member is such as to have a surface which diffusely reflects light from the light source.

This can help to ensure that the object and/or object manipulation member stand out against the background in the image since diffusely reflected light will tend not to be blocked by the elliptical polarisation filter.

The invention is defined in the appended independent claim.

The present invention will now be described, by way of example only, with reference to the accompanying drawings in which:
Figure 1 schematically shows an object handling apparatus, not claimed as such, comprising an image acquisition and processing apparatus;
Figure 2 shows an alternative object handling apparatus in the form of a robotic surgery apparatus;
Figure 3 schematically shows an image processing assisted surgery apparatus apparatus, not claimed as such, and;
Figure 4 shows a strain measurement apparatus, not claimed as such.

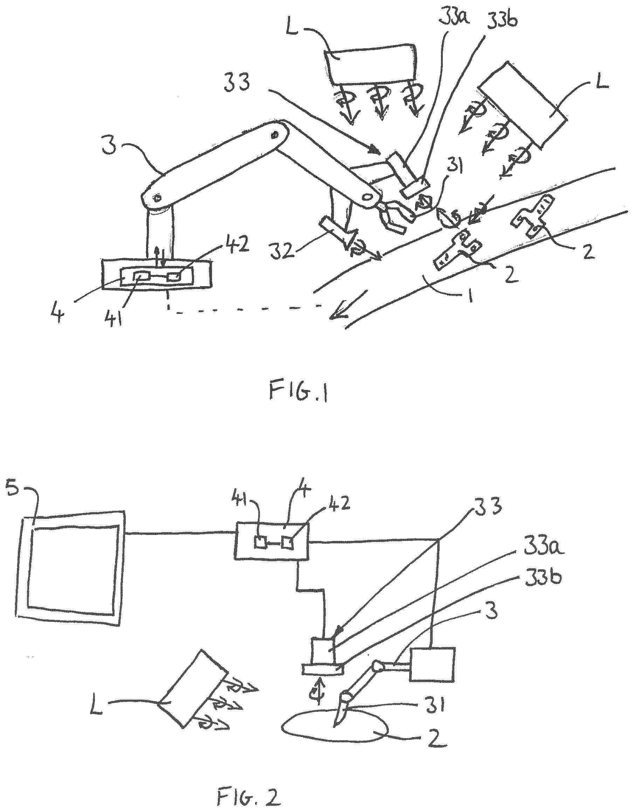

Figure 1 schematically shows an object handling apparatus, not claimed as such, which includes image acquisition and processing apparatus. The object handling apparatus comprises a conveyor arrangement, in this case a conveyor belt 1 on which are supported and transported a plurality of objects 2. The conveyor belt is an example of an object manipulation member. The apparatus further comprises a robot arm 3 comprising manipulator members 31 (for example, jaws) which may be arranged for gripping or performing a process on a selected one of the objects 2. Thus these are further object manipulation members.

Of course such object handling apparatus in general are known. However, difficulties can arise in their operation - particularly reliability issues due to having inadequate or inaccurate information concerning the location of objects in the apparatus. An aim of the present techniques is to seek to improve this.

In this example the robot arm 3 also carries a light source arrangement 32 and a light detector arrangement 33 comprising a camera 33a. Further additional light source arrangements L are provided and together with the light source 32 on the arm 3 these illuminate the object area in which the conveyor 1 and the plurality objects 2 are located.

Each of the light source arrangements L, 32 is arranged for illuminating the object area with elliptically polarised, in this case circularly polarised, light with a first chirality. Thus in this example, each of the light source arrangements is arranged to illuminate the area with clockwise polarised light.

Correspondently, the light detector arrangement 33 comprises a filter 33b for allowing passage of clockwise polarised light to the camera 33a but blocking anti-clockwise polarised light.

The robot arm 3 further comprises a central unit 4 which comprises image processing means 41 and a control unit 42 for controlling the robot arm in dependence on the output from the image processing means 41. In this example, the control unit 42 is arranged for also controlling the operation of the conveyor belt 1.

The specific details of the image processing means 41 and control unit 42 are not described in detail in the present specification since these details are not directly relevant to the present invention. Existing standard techniques may be used in image processing and control in the operation of the present invention.

Important to the present techniques is the use of light source arrangements 32, L which are arranged for illuminating the area with elliptically polarised light and using the light detector arrangement 33 arranged for allowing light with the same elliptical polarisation to be accepted into the detector system but blocking light with the opposite elliptical polarity. This can provide enhanced performance by reducing unwanted image artefacts due to specular reflection. As explained above this is because specular reflection from the objects 2 and other items in the object area will reverse the elliptical polarisation of the illuminating light and, as such, only diffusely reflected light will tend to make its way into the detecting system in the present systems.

Thus in operation of the object handling apparatus of Figure 1, the object area is illuminated by the light source arrangements L, 32 and images are captured by the camera 33a which are enhanced by virtue of the suppression of unwanted reflections as explained above. This image data is then fed to the central unit 4 where it may be processed by the image processing means 41 and decisions made in the control unit 42 in dependence on those processed images for controlling the operation of the robot arm 3 and in particular the object manipulation members 31 as well as operation of the conveyor 1.

Thus, in particular, an image captured by the camera 33a may enable determination of the position of one of the objects 2 relative to the object manipulation members 31 and following this determination, appropriate control signals may be sent to the conveyor 1 and/or the robot arm 3 in order to move the object 2 manipulation members 31 relative to the object 2 such that the object may be held and/or processed by the object manipulation members 31.

Note that in the present system the image detector arrangement 33 in particular the camera 33a is carried by the robot arm 3. Similarly one light source arrangement 32 is carried by the robot arm 3. In some cases the other static light source arrangements L might be removed. In the present system with the use of elliptically polarised light (in particular, circularly polarised light), suppression of reflection based artefacts may be obtained even though the relative position and orientation of the camera 33a and light source 32 compared to each other and/or the object 2 may be changed or changing to significant degree.

To put this another way, the present system allows image enhancement to be achieved in systems with multiple degrees of freedom and further allows direct and accurate illumination and imaging by virtue of facilitating the light source and camera to be carried on the robot arm or, more generally, to be moved together with a respective object manipulation member 31.

In more general terms, the use of elliptical polarisation (and in particular circular polarisation) overcomes restrictions which will be incurred with systems making use of linear polarised lighting and simplifies the set up. In turn this enables cameras, lighting, and objects under view to be moved freely within the object area without introducing unwanted reflective artefacts or adversely affecting the contrast needed to process the images acquired.

Figure 2 shows robotic surgery apparatus. This apparatus is similar in some respects to the apparatus described above in relation to Figure 1. Again there is a robot arm 3 carrying an object manipulation member 31, in this case a surgical instrument. Again a light source arrangement L for illuminating the area with circularly polarised light is provided and a camera 33a is provided for acquiring images together with a filter 33b for blocking light with a circular polarisation which is opposite to that provided by the light source arrangement L.

As will be appreciated whilst the light source arrangement L and camera 33a are shown separately from the robot arm 3 and as such are static components in the arrangement of Figure 2, in other embodiments a light source might be provided on the robot arm 3 or otherwise be mobile and similarly the camera 33a might be provided on the robot arm 3 or otherwise be mobile.

Again a central unit 4 comprising image processing means 41 and a control unit 42 are provided for accepting image data from the camera 33, processing that data and outputting suitable control signals to the robot arm 3 as well as outputting an image to a display 5 for viewing by a user.

Again in this system, reduction in reflection artefacts can assist in the decision making required to determine the control signals.

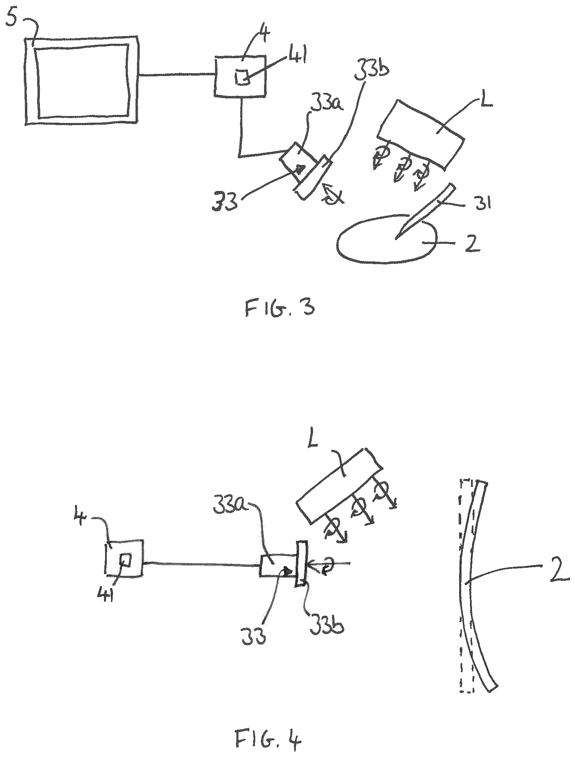

Figure 3 shows an image processing assisted surgery apparatus, not claimed as such, which is similar to the apparatus in Figure 2 but which does not include a robotic arm. Here the object manipulation member 31 is again a surgical instrument but in this case that will be held and used by a user i.e. a surgeon. Here the system is used to acquire and display to the user images which have been enhanced by virtue of the current techniques.

Thus again the object area is illuminated by circularly polarised light and a camera 33a is provided for acquiring images whilst having a filter 33b for blocking light having a circular polarisation which is opposite to that of the light source arrangement L. Again a central unit 4 is provided and this includes an image processing means 41 for accepting the image data from the camera 33a and providing this to a display 5 for viewing by a user.

Figure 4 shows a strain measurement apparatus, not claimed as such, which comprises an image acquisition and processing apparatus which is similar to that described above.

Here the object 2 observed is a sample whose strain is to be measured. The apparatus is arranged for detecting changes in size/shape of the object in order to determine the amount of strain to which the object has been subjected. Again a light source arrangement L for providing circularly polarised light to illuminate the object area is provided. Similarly a camera 33a and a filter 33b for blocking circularly polarised light with an opposite polarisation to that which is supplied by the light source L is provided for acquiring an image of the object 2. The output of the camera 33a is fed to a central unit 4 which comprises image processing means 41 for allowing a determination of changes in size/shape of the object 2 and hence the determination of the strain to which the object 2 has been subjected. For example correlation techniques may be used.

In some cases, the strain measurement apparatus may form part of a testing system where the object 2 is deliberately subjected to load in order to cause strain and in such as a case the apparatus will include suitable loading means for subjecting the object 2 to strain.

In each of the above cases, the use of circularly polarised light and a filter 33b on the camera 33a which blocks light with an opposite circular polarisation helps reduce image artefacts due to specular reflection and at the same time allows flexibility in the set up.

Thus, for example, in the strain measurement apparatus shown in Figure 4 the initial set up of the system will be simpler even if it is a relatively static system. However the use of a static system is not a requirement. That is to say, strain of an object which may be moving translationally or in terms of orientation in the object area may also be monitored and measured. Similarly in the case of the two medical examples shown in Figures 2 and 3 the fact that the object 2, in this case live tissue, being operated on may be moving and changing the orientation between itself and the light source and/or camera does not stop the image enhancement being achieved.

## Claims

1. An object handling apparatus comprising a surgical robot having a robotic arm (3) and further comprising image acquisition and processing apparatus arranged for determining positional information in respect of an object area occupied by an object (2), the apparatus comprising:
a light source arrangement (L) for illuminating the object area;
a light detector arrangement (33a) for detecting light reflected from the object area;
image processing means (41) arranged for determining positional information in respect of the object (2) in dependence on output from the light detector arrangement (33a);
a control unit (42) arranged for controlling the robotic arm (3) in dependence on the output from the image processing means (41);
wherein the light source arrangement (L) is arranged for illuminating the object area with elliptically polarised light with a first chirality; and the light detector arrangement (33a) comprises an elliptical polarisation filter (33b) for allowing passage of elliptically polarised light with said first chirality and blocking elliptically polarised light with an opposite chirality for suppressing generation of image artefacts due to specular reflection of the elliptically polarised light from the object area, and
wherein the robotic arm (3) comprises an object manipulation member (31) provided in the object area for manipulating the object (2), the object manipulation member (31) having a surface which diffusely reflects light from the light source (L).

2. An object handling apparatus according to claim 1 in which the image processing means (41) is arranged for determining positional information in respect of the object manipulation member (31).

3. An object handling apparatus according to claim 1 or 2 in which the image processing means (41) is arranged for determining the position of the object (2) relative to the object manipulation member (31).

4. An object handling apparatus according to any preceding claim comprising control means (42) arranged to make decisions and/or control components in dependence on output from the light detector arrangement (33a).

5. An object handling apparatus according to any preceding claim comprising control means (42) for controlling the position of the object manipulation member (31) in the object area in dependence on output from the light detector arrangement (33a).

6. An object handling apparatus according to any preceding claim which comprises a plurality of object manipulation members and the control means (42) is arranged for controlling the position of each of the object manipulation members in dependence on output from the light detector arrangement (33a).

7. An object handling apparatus according to any preceding claim in which the object (2) is such as to have a surface which diffusely reflects light from the light source.

## Patentansprüche

1. Objekthandhabungsvorrichtung, die einen chirurgischen Roboter mit einem Roboterarm (3) und ferner eine Bilderfassungs- und -verarbeitungsvorrichtung umfasst, die zum Bestimmen von Positionsinformationen in Bezug auf einen von einem Objekt (2) eingenommenen Objektbereich ausgelegt ist, wobei die Vorrichtung Folgendes umfasst:
eine Lichtquellenanordnung (L) zum Beleuchten des Objektbereichs;
eine Lichtdetektoranordnung (33a) zum Detektieren von Licht, das vom Objektbereich reflektiert wird;
Bildverarbeitungsmittel (41), ausgelegt zum Bestimmen von Positionsinformationen in Bezug auf das Objekt (2) in Abhängigkeit vom Ausgang der Lichtdetektoranordnung (33a);
eine Steuereinheit (42), ausgelegt zum Steuern des Roboterarms (3) in Abhängigkeit vom Ausgang des Bildverarbeitungsmittels (41);
wobei die Lichtquellenanordnung (L) zum Beleuchten des Objektbereichs mit elliptisch polarisiertem Licht mit einer ersten Chiralität ausgelegt ist; und die Lichtdetektoranordnung (33a) ein elliptisches Polarisationsfilter (33b) aufweist, um elliptisch polarisiertes Licht mit der genannten ersten Chiralität durchzulassen und elliptisch polarisiertes Licht mit einer entgegengesetzten Chiralität zu blockieren, um die Erzeugung von Bildartefakten aufgrund von Spiegelreflexion des elliptisch polarisierten Lichts von dem Objektbereich zu unterdrücken, und
wobei der Roboterarm (3) ein Objektmanipulationselement (31) umfasst, das im Objektbereich zum Manipulieren des Objekts (2) vorgesehen ist, wobei das Objektmanipulationselement (31) eine Oberfläche aufweist, die Licht von der Lichtquelle (L) diffus reflektiert.

2. Objekthandhabungsvorrichtung nach Anspruch 1, bei der das Bildverarbeitungsmittel (41) zum Bestimmen von Positionsinformationen in Bezug auf das Objektmanipulationselement (31) ausgelegt ist.

3. Objekthandhabungsvorrichtung nach Anspruch 1 oder 2, bei der das Bildverarbeitungsmittel (41) zum Bestimmen der Position des Objekts (2) relativ zu dem Objektmanipulationselement (31) ausgelegt ist.

4. Objekthandhabungsvorrichtung nach einem vorherigen Anspruch mit Steuermitteln (42), ausgelegt zum Treffen von Entscheidungen, und/oder Steuerkomponenten in Abhängigkeit vom Ausgang der Lichtdetektoranordnung (33a).

5. Objekthandhabungsvorrichtung nach einem vorherigen Anspruch mit Steuermitteln (42) zum Steuern der Position des Objektmanipulationselements (31) im Objektbereich in Abhängigkeit vom Ausgang der Lichtdetektoranordnung (33a).

6. Objekthandhabungsvorrichtung nach einem vorherigen Anspruch mit mehreren Objektmanipulationselementen, wobei das Steuermittel (42) zum Steuern der Position jedes der Objektmanipulationselemente in Abhängigkeit vom Ausgang der Lichtdetektoranordnung (33a) ausgelegt ist.

7. Objekthandhabungsvorrichtung nach einem vorherigen Anspruch, bei der das Objekt (2) so beschaffen ist, dass es eine Oberfläche aufweist, die das Licht von der Lichtquelle diffus reflektiert.

## Revendications

1. Un appareil de manipulation d'objets comprenant un robot chirurgical possédant un bras robotique (3) et comprenant en outre un appareil d'acquisition et de traitement d'image agencé de façon à déterminer des informations de position par rapport à une zone d'objet occupée par un objet (2), l'appareil comprenant :
un agencement de source lumineuse (L) destiné à éclairer la zone d'objet,
un agencement de détecteur de lumière (33a) destiné à la détection d'une lumière réfléchie à partir de la zone d'objet,
un moyen de traitement d'image (41) agencé de façon à déterminer des informations de position par rapport à l'objet (2) en fonction d'une sortie de l'agencement de détecteur de lumière (33 a),
une unité de commande (42) agencée de façon à commander le bras robotique (3) en fonction de la sortie du moyen de traitement d'image (41),
où l'agencement de source lumineuse (L) est agencé de façon à éclairer la zone d'objet avec une lumière à polarisation elliptique avec une première chiralité, et l'agencement de détecteur de lumière (33a) comprend un filtre à polarisation elliptique (33b) destiné à permettre le passage d'une lumière à polarisation elliptique avec ladite première chiralité et à bloquer une lumière à polarisation elliptique avec une chiralité opposée de façon à supprimer la génération d'artéfacts d'image dus à une réflexion spéculaire de la lumière à polarisation elliptique à partir de la zone d'objet, et
où le bras robotique (3) comprend un élément de manipulation d'objets (31) placé dans la zone d'objet destiné à la manipulation de l'objet (2), l'élément de manipulation d'objets (31) possédant une surface qui réfléchit de manière diffuse la lumière provenant de la source lumineuse (L).

2. Un appareil de manipulation d'objets selon la Revendication 1 dans lequel le moyen de traitement d'image (41) est agencé de façon à déterminer des informations de position par rapport à l'élément de manipulation d'objets (31).

3. Un appareil de manipulation d'objets selon la Revendication 1 ou 2 dans lequel le moyen de traitement d'image (41) est agencé de façon à déterminer la position de l'objet (2) par rapport à l'élément de manipulation d'objets (31).

4. Un appareil de manipulation d'objets selon l'une quelconque des Revendications précédentes comprenant un moyen de commande (42) agencé de façon à prendre des décisions et/ou des composants de commande en fonction d'une sortie de l'agencement de détecteur de lumière (33a).

5. Un appareil de manipulation d'objets selon l'une quelconque des Revendications précédentes comprenant un moyen de commande (42) destiné à la commande de la position de l'élément de manipulation d'objets (31) dans la zone d'objet en fonction d'une sortie de l'agencement de détecteur de lumière (33a).

6. Un appareil de manipulation d'objets selon l'une quelconque des Revendications précédentes qui comprend une pluralité d'éléments de manipulation d'objets, et le moyen de commande (42) est agencé de façon à commander la position de chacun des éléments de manipulation d'objets en fonction d'une sortie de l'agencement de détecteur de lumière (33a).

7. Un appareil de manipulation d'objets selon l'une quelconque des Revendications précédentes dans lequel l'objet (2) est tel qu'il possède une surface qui réfléchit de manière diffuse la lumière provenant de la source lumineuse.
